# EUROPÄISCHE PATENTANMELDUNG

(11) **EP 3 124 592 A1**
(43) Veröffentlichungstag der Anmeldung: **01.02.2017**
(21) Anmeldenummer: 16181249.0
(22) Anmeldetag: 26.07.2016
(51) Int. Cl.: C12M 1/107, C12M 1/00, C12M 1/34

(54) **VERFAHREN ZUR ANAEROBEN FERMENTATION VON BIOGENEN ABFALLSTOFFEN UND ANLAGE ZUR DURCHFÜHRUNG DIESES VERFAHRENS**

(30) Priorität: 31.07.2015 EP 15179224
(71) Anmelder: Hitachi Zosen Inova AG, 8005 Zürich (CH)
(72) Erfinder: SCHATZ, Adrian, 3172 Niederwangen (CH); LEISNER, René, 78467 Konstanz (DE)
(74) Vertreter: Schaad, Balass, Menzl & Partner AG

(57) **Zusammenfassung**

Die vorliegende Erfindung betrifft ein Verfahren zur anaeroben Fermentation von biogenen Abfallstoffen umfassend die Schritte, dass
a) ein die Abfallstoffe enthaltendes Fermentationsgut (B) mit Flüssigkeit gemischt wird und die derart erhaltene Fermentationsgutsuspension (BS) in einem Fermenter (1) anaerob fermentiert wird und
b) das bei der Fermentation der Fermentationsgutsuspension (BS) anfallende Digestat (D) mittels einer Entwässerungsvorrichtung (13) entwässert wird, wobei unter Abscheidung von Presswasser (PW) ein Presskuchen (PK) mit einem gegenüber dem Digestat (D) erhöhten Trockensubstanzanteil erhalten wird.

Das erfindungsgemässe Verfahren umfasst die zusätzlichen Schritte, dass
c) das Presswasser (PW) einer Feststoffabtrennung unterzogen wird, wobei eine Restflüssigkeit (RF; F) mit einem gegenüber dem Presswasser (PW) reduzierten Trockensubstanzanteil von höchstens 15 Gew.-% erhalten wird und
d) die Restflüssigkeit (RF; F) mindestens teilweise für das Mischen gemäss a) verwendet wird.

## Beschreibung

Die Erfindung betrifft ein Verfahren zur anaeroben Fermentation von biogenen Abfallstoffen gemäss Oberbegriff des Anspruchs 1 sowie eine Anlage zur Durchführung dieses Verfahrens gemäss Oberbegriff des Anspruchs 11.

Verfahren zur anaeroben Fermentation von biogenen Abfallstoffen, insbesondere von Haus-, Garten-, Landwirtschafts- und Industrieabfällen, sind dem Fachmann bekannt. Solche Verfahren erlauben einerseits eine ökologische Abfallbewirtschaftung, andererseits sind sie angesichts der bedeutenden Menge an Biogas, die damit erzeugt werden können, auch aus ökonomischer Sicht interessant.

Die anaeroben Fermentationsverfahren haben sich sowohl in der Massen- als auch in der Energiebilanz den aeroben als überlegen erwiesen. Entsprechende Anlagen für die anaerobe Fermentation benötigen verhältnismässig wenig Raum und können geruchsneutral betrieben werden, sodass für solche Anlagen auch Standorte in dicht besiedelten Gebieten in Frage kommen.

Unter den bekannten anaeroben Fermentationsverfahren können grundsätzlich Flüssig- oder Nass-Gärverfahren von den sogenannten Trocken-Gärverfahren unterschieden werden:

Nass-Gärverfahren werden üblicherweise in einem oder mehreren vertikal ausgerichteten Fermentern durchgeführt.

Ein entsprechendes Verfahren und eine entsprechende Anlage wird etwa in der GB 2 230 004 beschrieben.

Nachteilig an solchen Verfahren ist, dass schwerere Bestandteile des Fermentationsguts im Fermenter sedimentieren können, bevor sie vollständig abgebaut sind. Mithin können aufgrund einer zu kurzen Verweilzeit unvollständig abgebaute Bestandteile in den Ausgangsbereich des Fermenters gelangen, was sowohl aus ökonomischer als auch aus hygienischer Sicht nachteilig ist.

Bei den sogenannten Trocken-Gärverfahren wird ein Fermentationsgut mit wesentlich höherem Trockensubstanzanteil fermentiert, wobei auch in diesem Fall das Fermentationsgut in keiner Weise trocken ist. Bei diesen Verfahren wird die Fermentation in der Regel in einem horizontal ausgerichteten Fermenter durchgeführt.

So wird etwa in EP 0 621 336 ein Verfahren beschrieben, in dem biogene Abfälle in einem horizontalen Fermenter im Pfropfenströmungsbetrieb mit kontrollierter Durchmischung anaerob fermentiert werden. Gegenüber den erwähnten Flüssig-Gärverfahren zeichnet sich das Verfahren gemäss EP 0 621 336 dadurch aus, dass das Fermentationsgut einen verhältnismässig geringen Trockensubstanzanteil aufweist und somit nur ein relativ geringes Volumen bearbeitet werden muss. Dies bringt die Vorteile mit sich, dass entsprechende Anlage vergleichsweise klein dimensioniert und relativ kostengünstig betrieben werden können. Insbesondere wird durch den hohen Gehalt an Trockensubstanz im Fermentationsgut das Volumen an anfallendem Abwasser reduziert.

Trotz dieser Vorteile hat sich in der Praxis gezeigt, dass bei einem zu hohen Trockensubstanzanteil das Fermentationsgut nicht in der gewünschten Weise bzw. nur mit relativ grossem Aufwand im Fermenter gefördert werden kann.

Diesem Nachteil kann prinzipiell dadurch Abhilfe geschaffen werden, dass das Fermentationsgut mit Wasser verdünnt wird; allerdings wird damit einer der Hauptvorteile der Trocken-Gärverfahren, nämlich die Reduzierung von Abwasser, mindestens zu einem grossen Teil eingebüsst.

Ein Verfahren, gemäss welchem zur Einstellung der gewünschten Homogenität und des gewünschten Trockensubstanzgehalts Wasser zum Fermentationsgut zugeführt wird, wird in EP 1 076 051 beschrieben. Gemäss diesem Verfahren kann nebst Frisch- oder Brauchwasser das bei der Entwässerung des fermentierten Materials gewonnene Presswasser für die Verdünnung eingesetzt werden.

Allerdings kann das bakteriell beladene Presswasser nur bedingt für die Verdünnung eingesetzt werden, weil bei einer Rückführung des nach wie vor einen relativ hohen Trockensubstanzanteil aufweisenden Presswassers zusätzlich bereits abgebautes Material in den Fermenter zurückgeführt wird, was die Energiebilanz des Prozesses insgesamt beeinträchtigt. Dies fällt insbesondere bei wiederholtem Rezirkulieren ins Gewicht, da sich dabei das bereits abgebaute Material anreichert bzw. aufkonzentriert wird. Aufgrund dieser Beschränkungen für die Verwendung von Presswasser ist der Bedarf an eingesetztem Frischwasser und somit auch die Menge an bei der Fermentation anfallendem Abwasser auch für die in der EP 1 076 051 beschriebene Technologie nach wie vor relativ hoch.

Aufgabe der vorliegenden Erfindung ist es somit, ein Verfahren zur anaeroben Fermentation von biogenen Abfallstoffen zur Verfügung zu stellen, welches eine gute Förderbarkeit des Fermentationsguts gewährleistet und welches es gleichzeitig erlaubt, die Menge an beim Verfahren anfallendem Abwasser zu minimieren.

Insbesondere sollen gemäss vorliegender Erfindung die Vorteile bekannter Trocken-Gärverfahren, d.h. ein relativ hoher Durchsatz des Fermentationsguts bei relativ geringer Dimensionierung der Anlage, beibehalten werden.

Die Erfindung wird gelöst durch das Verfahren gemäss Anspruch 1. Bevorzugte Ausführungsformen der Erfindung werden in den abhängigen Ansprüchen definiert.

Gemäss Anspruch 1 betrifft die Erfindung ein Verfahren zur anaeroben Fermentation von biogenen Abfallstoffen umfassend die Schritte, dass
a) ein die Abfallstoffe enthaltendes Fermentationsgut mit Flüssigkeit gemischt wird und die derart erhaltene Fermentationsgutsuspension in einem Fermenter anaerob fermentiert wird und
b) das bei der Fermentation der Fermentationsgutsuspension anfallende Digestat mittels einer Entwässerungsvorrichtung entwässert wird, wobei unter Abscheidung von Presswasser ein Presskuchen mit einem gegenüber dem Digestat erhöhten Trockensubstanzanteil erhalten wird.

Gemäss der Erfindung umfasst das Verfahren nun die zusätzlichen Schritte, dass
c) das Presswasser einer Feststoffabtrennung unterzogen wird, wobei eine Restflüssigkeit mit einem gegenüber dem Presswasser reduzierten Trockensubstanzanteil von höchstens 15 Gew.-% erhalten wird, und
d) die Restflüssigkeit mindestens teilweise für das Mischen gemäss a) verwendet wird.

Im Gegensatz zu den im Stand der Technik bekannten Verfahren wird somit aus dem bei der Entwässerung erhaltenen Presswasser vor dessen Rückführung in den Fermenter ein weiterer Feststoffanteil abgetrennt und der Trockensubstanzanteil derart auf einen Bereich mit einem definierten Höchstwert von 15 Gew.-% gesenkt.

Feststoffabtrenner, welche hierfür zum Einsatz kommen können, werden weiter unten spezifiziert. Gemäss einer besonders bevorzugten Ausführungsform wird für die Feststoffabtrennung gemäss Schritt c) ein Schwingsieb und/oder ein Rüttelsieb verwendet.

Mithin wird gemäss der vorliegenden Erfindung ein bedeutender Anteil des aus dem Fermenter ausgeführten Wassers nach entsprechender Aufarbeitung wieder in den Fermenter zurückgeführt, wodurch einerseits der Bedarf an einzuführendem Frischwasser und andererseits die Menge an zu entsorgendem Abwasser reduziert werden kann.

Im Kontext der vorliegenden Erfindung wird derjenige Anteil an Flüssigkeit, der der Fermentationssuspension im Entwässerungsschritt entzogen wird, als Presswasser bezeichnet, und zwar unabhängig davon, ob die Flüssigkeit tatsächlich durch aktives Pressen oder durch passives Abtropfen vom Digestat abgetrennt wird.

Wie bereits im Zusammenhang mit dem technologischen Hintergrund der Erfindung erwähnt weist das Presswasser einen relativ hohen Gehalt an organischem Material auf und ist in der Regel schlammartig. Der Trockensubstanzanteil des Presswassers liegt in der Regel in einem Bereich von 15 bis 20 Gew.-%, kann aber je nach Art der Entwässerung bzw. der hierfür eingesetzten Entwässerungsvorrichtung etwas tiefer liegen. Als Entwässerungsvorrichtung kann beispielsweise eine Schneckenpresse eingesetzt werden.

Dadurch, dass das Presswasser erfindungsgemäss einer Feststoffabtrennung unterzogen wird, in welchem eine Restflüssigkeit mit einem gegenüber dem Presswasser reduzierten Trockensubstanzanteil von höchstens 15 Gew.-% erhalten wird, kann gewährleistet werden, dass beim nachfolgenden Mischen der Restflüssigkeit mit dem Fermentationsgut eine optimal förderbare Fermentationsgutsuspension erhalten wird und dass gleichzeitig die Rückführung von energetisch minderwertigem Material minimiert wird.

Wie das Presswasser besteht somit auch die Restflüssigkeit aus einer wässrigen Suspension, wobei aber der Anteil an Trockensubstanz, welcher hauptsächlich aus organischen Schwebestoffen besteht, in der Restflüssigkeit geringer ist als im Presswasser. Wie erwähnt liegt der Trockensubstanzanteil der Restflüssigkeit bei höchstens 15 Gew.-%, wobei er bevorzugt in einem Bereich von 5 bis 15 Gew.-% liegt, besonders bevorzugt in einem Bereich von 5 bis 10 Gew.-%. Gemäss der bevorzugtesten Ausführungsform beträgt der Trockensubstanzanteil mehr als 5 Gew.-% und weniger als 10 Gew.-%.

Um den Trockensubstanzanteil auf den gemäss Schritt c) definierten Höchstwert von 15 Gew.-% zu senken, reichen konventionelle Entwässerungsvorrichtungen nicht aus, weshalb zusätzlich ein Feststoffabtrenner zur Anwendung kommt. Wie erwähnt wird für die Feststoffabtrennung bevorzugt ein Sieb verwendet, welches gemäss einer besonders bevorzugten Ausführungsform mindestens zwei Siebstufen aufweist. Diesbezüglich ist weiter bevorzugt, dass die Feststoffabtrennung unter Verwendung eines Schwingsiebs und/oder eines Rüttelsiebs erfolgt.

Als "Trockensubstanzanteil" wird im Kontext der vorliegenden Erfindung der Gewichtsanteil der Trockensubstanz bzw. das Gewicht der Trockensubstanz bezogen auf das Gesamtgewicht des jeweiligen Materials bezeichnet. Methoden zur Bestimmung des Trockensubstanzanteils sind dem Fachmann bekannt und können etwa derart durchgeführt werden, dass die flüssigen Anteile des Materials in einem Ofen abgezogen werden und danach das Gewicht der verbleibenden Trockensubstanz bestimmt wird.

Die Rückführung der Restflüssigkeit hat erfindungsgemäss in erster Linie den Zweck, das Fermentationsgut zu verdünnen bzw. eine optimal förderbare Fermentationsgutsuspension bereitzustellen. Die Erfindung unterscheidet sich somit grundlegend von dem in der EP 1 095 924 dargelegten Verfahren, gemäss welchem Presswasser, welches einen relativ hohen Trockensubstanzanteil aufweist und bakteriell beladen ist, als Impfmaterial für die zu vergärenden Abfallstoffe verwendet wird. Vielmehr wird für das Animpfen gemäss einer bevorzugten Ausführungsform der Erfindung ein Teil des Digestats, des in Schritt b) erhaltenen Presskuchens, des in Schritt c) abgetrennten Restfeststoffs oder einer Mischung davon verwendet, wie weiter unten ausgeführt wird.

Wie in der EP 1 095 924 wird auch in der DE 10 2005 012 367 eine Feststoffabtrennung zum Erhalt einer Restflüssigkeit mit einem gegenüber dem Presswasser reduzierten Trockensubstanzanteil von höchstens 15 Gew.-% nicht offenbart. Vielmehr wird auch in der DE 10 2005 012 367 das dabei anfallende Prozesswasser zum Anmaischen bzw. Animpfen verwendet, was - wie im Falle der EP 1 095 924 - einen relativ hohen Trockensubstanzanteil erfordert.

Wie ebenfalls weiter unten detailliert dargelegt wird, erfolgt die Rückführung der Restflüssigkeit für das Mischen gemäss Schritt a) entweder direkt oder indirekt, insbesondere über einen oder mehrere Tanks zur Zwischenlagerung der Restflüssigkeit.

In der Regel wird das erfindungsgemässe Verfahren kontinuierlich oder quasi-kontinuierlich durchgeführt, womit es sich zusätzlich von chargenweise betriebenen Verfahren unterscheidet, wie sie etwa in der GB 2 230 004 offenbart werden. Als "quasi-kontinuierlich" werden dabei Verfahren bezeichnet, die an sich kontinuierlich betrieben werden, bei denen die Zugabe von Fermentationsgut aber nicht ununterbrochen erfolgt, sondern in Zeitintervallen.

Zudem wird die Fermentation im erfindungsgemässen Verfahren - in weiterem Unterscheid zur GB 2 230 004 - vorzugsweise in einem horizontal ausgerichteten Fermenter durchgeführt, wie ebenfalls weiter unten ausgeführt wird.

Gemäss einer besonders bevorzugten Ausführungsform erfolgt die Feststoffabtrennung gemäss Schritt c) mittels Filtration und/oder mittels Sieben. Die Restflüssigkeit wird gemäss dieser Ausführungsform somit durch das Filtrat der Filtration bzw. den Siebdurchgang des Siebens gebildet.

Weiter kann bevorzugt sein, das in Schritt b) erhaltene Presswasser und/oder die in Schritt c) erhaltene Restflüssigkeit, insbesondere das Filtrat, vor dem jeweils nachfolgenden Schritt zwischenzulagern. Dies erlaubt es, die Menge an zuzugebender Restflüssigkeit genau und zeitnah zuzudosieren und somit an das vorliegende Fermentationsgut bzw. dessen Trockensubstanzanteil anzupassen, was gerade angesichts der Inhomogenität des frisch zugeführten Fermentationsguts von Bedeutung ist.

In der Regel wird das Fermentationsgut mit der Restflüssigkeit in einer Mischvorrichtung gemischt, bevor die derart erhaltene Fermentationsgutsuspension in den Fermenter eingeführt wird. Gemäss dieser Ausführungsform wird somit die Fermentationsgutsuspension ausserhalb des Fermenters vorgemischt, bevor sie in den Fermenter eingeführt wird. Alternativ dazu ist auch möglich, dass die Mischung im Fermenter erfolgt, insbesondere, dass die Mischvorrichtung ein Teil des Fermenters darstellt.

Je nach Bedarf kann zusätzlich zur verwendeten Restflüssigkeit optional weiteres Wasser zugeführt werden, was im Kontext der vorliegenden Erfindung in Abgrenzung zum rezyklierten Wasser der Restflüssigkeit als Frischwasser bezeichnet wird.

Vorzugsweise wird die Verdünnung des Fermentationsguts gemäss Schritt a) derart durchgeführt, dass der Trockensubstanzanteil in der Fermentationsgutsuspension zwischen 25 und 35 Gew.-% beträgt. Der Flüssigkeitsanteil ist somit ausreichend hoch, um eine optimale Förderbarkeit zu gewährleisten. Gleichzeitig ist der Trockensubstanzanteil ausreichend hoch, um den Fermentationsgutdurchsatz zu maximieren.

Wie erwähnt dient die Rückführung der Restflüssigkeit, insbesondere des Filtrats, in erster Linie der Verdünnung des Fermentationsguts, nicht aber dem Animpfen. Das Animpfen wird vorzugweise über teilweise Rückführung des Digestats, des bei der Entwässerung gemäss Schritt b) erhaltenen Presskuchens und/oder des bei der Feststoffabtrennung in Schritt c) erhaltenen Restfeststoffs als Impfmaterial erzielt. Diesbezüglich ist auch denkbar, eine Mischung von mindestens zwei dieser Fraktionen als Impfmaterial zurückzuführen.

Obschon der Zweck der Rückführung der Restflüssigkeit, insbesondere des Filtrats, in erster Linie der Verdünnung dient, kann es unter Umständen angezeigt sein, eine oder mehrere der folgenden Parameter zu bestimmen und ausgehend davon auf einen gewünschten Wert einzustellen:
- pH-Wert,
- Pufferkapazität,
- H₂S-Gehalt,
- Ammoniumionen- oder Ammoniak-Gehalt,
- Gehalt an Fettsäuren und
- Gehalt an kurzkettigen Carbonsäuren

Um den gewünschten Wert zu erhalten kann es somit bevorzugt sein, der Restflüssigkeit folgende Hilfs- oder Zusatzstoffe allein oder im Gemisch beizugeben:
- Säure,
- Lauge,
- Puffer,
- Mikronährelemente oder Spurenelemente und
- Makronährelemente.

Durch diese Einstellung verschiedener Parameter der Restflüssigkeit kann die Fermentationsgutsuspension somit optimal auf die im Fermenter ablaufenden Fermentationsprozesse abgestimmt werden.

Wie erwähnt kann die Feststoffabtrennung gemäss Schritt c) mittels Filtration durchgeführt werden. Diesbezüglich hat sich gezeigt, dass die Filtration mit einer vertikal ausgerichteten Filtervorrichtung durchgeführt werden kann, die eine hohlzylindrische Filtersäule mit einer perforierten Zylindermantelfläche, eine der Filtersäule konzentrisch angeordnete, sich axial um die Zylinderachse drehende Förderschnecke sowie eine ausserhalb des Filterzylinders angeordnete Filtratkammer umfasst, in welcher ein Unterdruck zum Ansaugen des Filtrats erzeugt wird. In dieser wird das Presswasser vom unteren Ende der Förderschnecke entlang der Innenfläche der Filtersäule nach oben in Richtung zu einem Feststoffauswurf gefördert und Filtrat durch die Filteröffnungen in den unter Unterdruck stehenden Filtratraum abgesogen. Ein Beispiel einer solchen Filtervorrichtung stellt etwa ein Wendel-Filter dar.

Hinsichtlich Effizienz und Eigenschaften des erhaltenen Filtrats ist allerdings besonders bevorzugt, dass die Feststoffabtrennung gemäss Schritt c) mittels eines Siebes, insbesondere mittels eines Schwingsiebs und/oder eines Rüttelsiebs erfolgt. In der Regel weist das Schwingsieb dabei zwei oder mehr Siebstufen auf, d.h. zwei Siebbeläge, die sich in der Maschenweite voneinander unterscheiden. Dabei wird in einer ersten Siebstufe c1) eine Grobfraktion an Feststoffbestandteilen vom Presswasser abgetrennt, und von der aus der Stufe c1) als Siebdurchgang verbleibenden Suspension in einer nachfolgenden zweiten Siebstufe c2) eine Feinfraktion abgetrennt, um die Restflüssigkeit zu erhalten. Typischerweise wird die Schwingbewegung des Siebbehälters bzw. der Siebbehälter mittels einer exzentrisch angeordneten Antriebswelle bzw. mittels einer exzentrisch auf einer Antriebswelle angeordneten Schwungmasse erhalten.

Es hat sich überraschenderweise gezeigt, dass sich mit dem beschriebenen Schwingsieb ein Trockensubstanzanteil von höchstens 15 Gew.-% in der Restflüssigkeit ohne weiteres erzielen lässt. Das gemäss dieser Ausführungsform zu verwendende Schwingsieb ist insbesondere einem unbewegten Membranfilter als Feststoffabtrenner überlegen, da einer Verstopfung des Siebbelags wirksam entgegengewirkt und damit auch ein kontinuierlicher Betrieb der Anlage gewährleistet werden kann. Im Übrigen werden hinsichtlich Effizienz des Verfahrens und Eigenschaften der Restflüssigkeit, insbesondere hinsichtlich dessen Trockensubstanzanteils, mit einem Schwingsieb sogar bessere Resultate erzielt als mit einem Wendel-Filter.

Nebst dem oben beschriebenen Verfahren betrifft die vorliegende Erfindung zudem eine Anlage zur Durchführung des Verfahrens.

In Analogie zu obiger Beschreibung des Verfahrens umfasst die Anlage
A) einen Fermenter zur Durchführung der anaeroben Fermentation und
B) eine mit dem Fermenter über eine Digestatzuleitung verbundene Entwässerungsvorrichtung zur Entwässerung des Digestats.

Gemäss der Erfindung umfasst die Anlage zudem
C) einen mit der Entwässerungsvorrichtung über eine Presswasserzuleitung verbundenen Feststoffabtrenner zur Abtrennung von Restfeststoff aus dem in der Entwässerungsvorrichtung erhaltenen Presswasser, wobei der Feststoffabtrenner derart ausgestaltet ist, dass die dabei erhaltene Restflüssigkeit einen Trockensubstanzanteil von höchstens 15 Gew.-% aufweist, und
D) eine Restflüssigkeitzuleitung zur mindestens teilweisen Rückführung der Restflüssigkeit in den Fermenter.

Wie ebenfalls oben erwähnt, ist der Feststoffabtrenner gemäss einer besonders bevorzugten Ausführungsform eine Filtrationsvorrichtung und/oder ein Sieb, welche(s) derart ausgestaltet ist, dass das dabei erhaltene Filtrat bzw. der dabei erhaltene Siebdurchgang einen Trockensubstanzanteil von höchstens 15 Gew.-% aufweist.

In Analogie zu den obigen, das erfindungsgemässe Verfahren betreffenden Ausführungen unterscheidet sich auch die erfindungsgemässe Anlage von derjenigen gemäss der EP-A-1 095 924, welche darauf ausgelegt ist, Presswasser als Impfmittel zu verwenden und somit keinen Feststoffabtrenner C) umfasst.

Weiter ist bevorzugt, dass der Fermenter der Anlage wenigstens annähernd horizontal ausgerichtet ist, womit er sich ganz offensichtlich von dem in GB 2 230 004 offenbarten Fermenter unterscheidet, welcher auf ein chargenweise betriebenes Verfahren ausgerichtet ist.

Weiter ist bevorzugt, dass die Restflüssigkeitszuleitung in eine Mischvorrichtung zum Mischen des Fermentationsguts mit Flüssigkeit mündet, welche über eine Speiseleitung mit dem Fermenter verbunden ist. Die Vormischung wird in dieser bevorzugten Ausführungsform der Anlage somit in einer vom Fermenter separaten Vorrichtung durchgeführt. Die Rückführung in den Fermenter erfolgt somit nicht direkt, sondern indirekt über die genannte Mischvorrichtung.

Für den oben genannten Fall, dass der Feststoffabtrenner eine Filtrationsvorrichtung ist, umfasst die Filtrationsvorrichtung vorzugsweise
C1) eine hohlzylindrische Filtersäule mit einer perforierten Zylindermantelfläche,
C2) eine in der Filtersäule konzentrisch angeordnete, sich axial um die Zylinderachse drehende Förderschnecke und
C3) eine ausserhalb des Filterzylinders angeordnete Filtratkammer, welcher Mittel zur Erzeugung eines Unterdrucks zum Ansaugen des Filtrats zugeordnet sind.

Wie oben beschrieben lassen sich durch eine solche Filtrationsvorrichtung, insbesondere durch einen Wendel-Filter, für die Zwecke der Erfindung besonders vorteilhafte Filtrate relativ einfach erhalten.

Die Erfindung wird anhand der Figuren weiter veranschaulicht. Von diesen zeigt:
- Fig. 1: ein Fliess-Schema des erfindungsgemässen Verfahrens;
- Fig. 2: eine schematische Darstellung einer erfindungsgemässen Anlage;
- Fig. 3: einen für die Zwecke der vorliegenden Erfindung besonders geeigneten Feststoffabtrenner in perspektivischer Ansicht;
- Fig. 4: den in Fig. 3 gezeigten Feststoffabtrenner in Seitenansicht; und
- Fig. 5: einen Schnitt durch den in Fig. 3 und 4 gezeigten Feststoffabtrenner durch die Schnittebenen A-A gemäss Fig. 4.

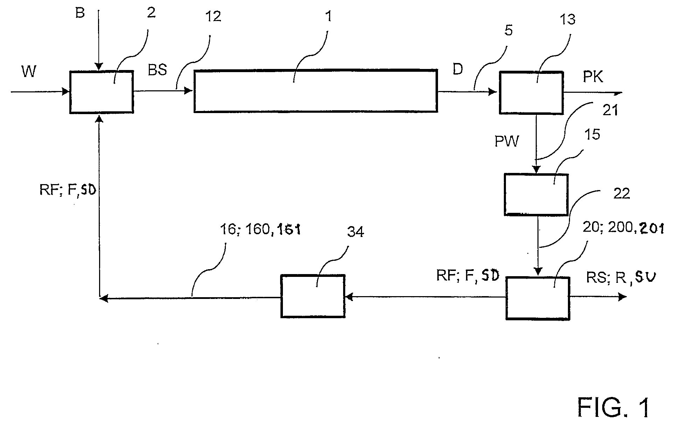

Das in Fig. 1 dargestellte konkrete Verfahren gemäss der vorliegenden Erfindung umfasst die Schritte, dass die anaerob zu fermentierenden biogenen Abfallstoffe als Fermentationsgut B in eine Mischvorrichtung 2 eingespeist werden.

In die Mischvorrichtung 2 wird Flüssigkeit eingeführt, um die gewünschte Homogenität und den gewünschten Trockensubstanzanteil der zu fermentierenden Fermentationsgutsuspension BS einzustellen. Mindestens ein Teil der zugeführten Flüssigkeit wird in Form eines Filtrats F zur Mischvorrichtung zugeführt, dessen Bereitstellung weiter unten im Detail beschrieben wird. Ein weiterer Teil der Flüssigkeit kann in Form von Frischwasser W zur Mischvorrichtung 2 zugeführt werden.

Die Mischvorrichtung kann etwa in analoger Weise ausgestaltet sein, wie in der EP-A-1 076 051 in den Absätzen [0020] und [0021] beschrieben, deren Inhalt hiermit unter Bezugnahme miteingeschlossen werden soll.

Die in der Mischvorrichtung 2 erzeugte Fermentationsgutsuspension BS weist einen Trockensubstanzanteil im Bereich von 25 bis 35 Gew.-% auf und wird über eine Speiseleitung 12 einem Fermenter 1 zugeführt. Die Zuführung erfolgt dabei kontinuierlich oder quasi-kontinuierlich und bestimmt die Durchlaufzeit der Fermentationsgutsuspension BS im Fermenter. Alternativ zur gezeigten Ausführungsform, in der die Mischvorrichtung 2 eine zum Fermenter 1 separate Vorrichtung darstellt, kann das Mischen des Fermentationsguts B mit zusätzlicher Flüssigkeit auch im Fermenter durchgeführt werden.

Das bei der Fermentation der Fermentationsgutsuspension BS anfallende und mindestens annähernd vollständig fermentierte Digestat D, dessen Trockensubstanzanteil im konkreten Fall noch im Bereich von 20 bis 30 Gew.-% liegt, wird schliesslich über eine Digestatzuleitung 5 einer Entwässerungsvorrichtung 13 zugeführt und mittels dieser Entwässerungsvorrichtung entwässert, wobei unter Abscheidung von Presswasser PW ein Presskuchen PK mit einem gegenüber dem Digestat D erhöhten Trockensubstanzanteil erhalten wird. Konkret liegt der Trockensubstanzanteil des Presskuchens PK im Bereich von 30 bis 40 Gew.-%, während der Trockensubstanzanteil des Presswassers PW im konkreten Fall zwischen 15 bis 20 Gew.-% liegt.

Das derart gewonnene Presswasser PW wird dann einem Feststoffabtrenner 20 in Form einer Filtrationsvorrichtung 200 oder eines Siebes 201 zugeführt, wobei als Restflüssigkeit RF ein Filtrat F bzw. ein Siebdurchgang SD mit einem gegenüber dem Presswasser PW reduzierten Trockensubstanzanteil von höchstens 15 Gew.-%, konkret einem Trockensubstanzanteil im Bereich von 5 bis 15 Gew.-% erhalten wird, während das als Restfeststoff RS der Filtration anfallende Retentat R bzw. der Siebüberstand SU einen Trockensubstanzanteil im Bereich von 35 bis 45 Gew.-% aufweist.

Wie bereits erwähnt wird schliesslich das Filtrat F bzw. der Siebdurchgang SD zur Mischvorrichtung 2 zurückgeführt, wo es zur Homogenisierung des Fermentationsguts B und zur Einstellung des erwünschten Trockensubstanzanteils verwendet wird.

In der konkret gezeigten Ausführungsform wird das bei der Entwässerung erhaltene Presswasser PW über einen ersten Presswasserzuleitungsabschnitt 21 einem Presswassertank 15 zugeführt, in dem es zwischengelagert wird, bevor es über einen zweiten Presswasserzuleitungsabschnitt 22 zur Filtrationsvorrichtung 200 geleitet wird. Zudem wird auch das bei der Filtration erhaltene Filtrat F in einem Filtrattank 34 zwischengelagert, bevor es über eine Restflüssigkeitzuleitung 16 in Form einer Filtratzuleitung 160 zur Mischvorrichtung 2 zurückgeführt wird. Selbstverständlich ist auch denkbar, dass das Presswasser PW bzw. das Filtrat F oder der Siebdurchgang SD direkt, d.h. ohne Zwischenlagerung, der nachfolgenden Filtrations- bzw. Mischvorrichtung 200 bzw. 2 zugeführt wird.

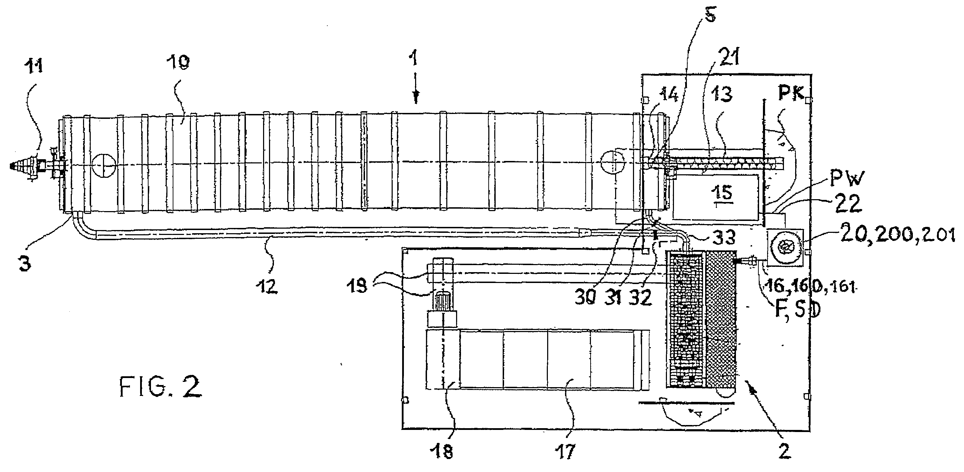

Die erfindungsgemässe Anlage wird im Folgenden anhand der Fig. 2 weiter erläutert.

In der in Fig. 2 konkret gezeigten Ausführungsform umfasst der Fermenter 1 einen horizontal ausgerichteten Fermentertank 10.

Der Fermentertank 10 kann entweder als Stahlkonstruktion oder als ausgekleidete Betonkonstruktion gestaltet sein und erstreckt sich in Achsrichtung von einem ersten einlassseitigen Ende 101 zu einem zweiten auslassseitigen Ende 102. Der Fermentertank 10 wird axial durch ein Rührwerk durchsetzt, welches dazu bestimmt ist, das über einen Fermentereinlass 3 eingeführte Fermentationsgut zu durchmischen, während es den Fermentertank 10 in Richtung zu einem Fermenterauslass 14 hin passiert. Dem Rührwerk ist ein Rührwerkantrieb 11 zugeordnet, welches am einlassseitigen Ende des Fermentertanks 10 angeordnet ist.

Die Einspeisung des Fermentationsguts erfolgt über eine vorzugsweise beheizbare Speiseleitung 12, welche einerseits am Fermentereinlass 3 angeschlossen ist und welche andererseits mit einer im Zusammenhang mit Fig. 1 beschriebenen Mischvorrichtung 2 in Verbindung steht, in der die einzuspeisende Fermentationsgutsuspension bereitgestellt wird.

Wie im Zusammenhang mit Fig. 1 erwähnt erfolgt die Einspeisung kontinuierlich beziehungsweise quasi-kontinuierlich. Ebenso erfolgt der Durchlauf durch den Fermenter 1 kontinuierlich bzw. quasi-kontinuierlich.

Über den Fermenterauslass 14 gelangt das wenigstens annähernd vollständig fermentierte Digestat in eine Entwässerungsvorrichtung 13 zum Entwässern des Digestats. Während der über die Entwässerung erhaltene Presskuchen PK einer Nachrotte zugeführt wird, wird das Presswasser PW über einen ersten Presswasserzuleitungsabschnitt 21 einem Presswassertank 15 zugeführt und dort zwischengelagert. Über einen zweiten Presswasserzuleitungsabschnitt 22 wird Presswasser vom Presswassertank 15 Feststoffabtrenner 20 in Form einer Filtervorrichtung 200 oder eines Siebes 201 zugeführt, in der das Presswasser filtriert wird, sodass das/der als Restflüssigkeit erhaltene Filtrat F bzw. Siebdurchgang SD einen Trockensubstanzanteil von höchstens 15 Gew.-% aufweist.

Von der Filtervorrichtung 23 wird das Filtrat F bzw. der Siebdurchgang SD über eine Restflüssigkeitszuleitung 16 in Form einer Filtratzuleitung 160 bzw. Siebdurchgangzuleitung 161 der Mischvorrichtung 2 zugeführt, welche zudem mit frischem zu behandelnden Fermentationsgut beschickt wird.

Die Mischvorrichtung 2 wird hier nicht wie üblich von einem gebäudeintern angeordneten Zwischenbunker beschickt, vielmehr gelangt das frische Fermentationsgut direkt von der Anlieferung auf einen Dosierförderer 17, von dem das Fermentationsgut über eine Austragseinheit 18 auf ein in die Mischvorrichtung mündendes Förderband 19 gelangt.

Üblicherweise werden die zu fermentierenden Abfallstoffe zerkleinert, bevor sie dem Fermenter zugeführt werden. Dazu können Zerkleinerer eingesetzt werden, die hier nicht dargestellt sind. Statt die Abfallstoffe auf eine geeignete Grösse zu zerkleinern, kann das Fermentationsgut auch durch Sieben, zum Beispiel in einem Trommelsieb, von zu grossen Bestandteilen befreit werden.

Die Mischvorrichtung 2 steht über eine Verbindungsleitung 33 mit einer Fermenterrückleitung 30 und andererseits mit der Speiseleitung 12 in Verbindung, wobei sich die Fermenterrückleitung 30 mittels eines Schiebers 31 öffnen und schliessen lässt. Auch der Speiseleitung 12 ist ein Schieber 32 zum Öffnen und Schliessen zugeordnet. Somit kann bei geöffnetem Schieber 31 Digestat aus dem Fermenter 1 in die Verbindungsleitung 33 eingebracht werden. Dieses Digestat gelangt somit wieder an das einlassseitige Ende des Fermentertanks, wo es als Impfmaterial für den Fermentationsprozess eingesetzt wird.

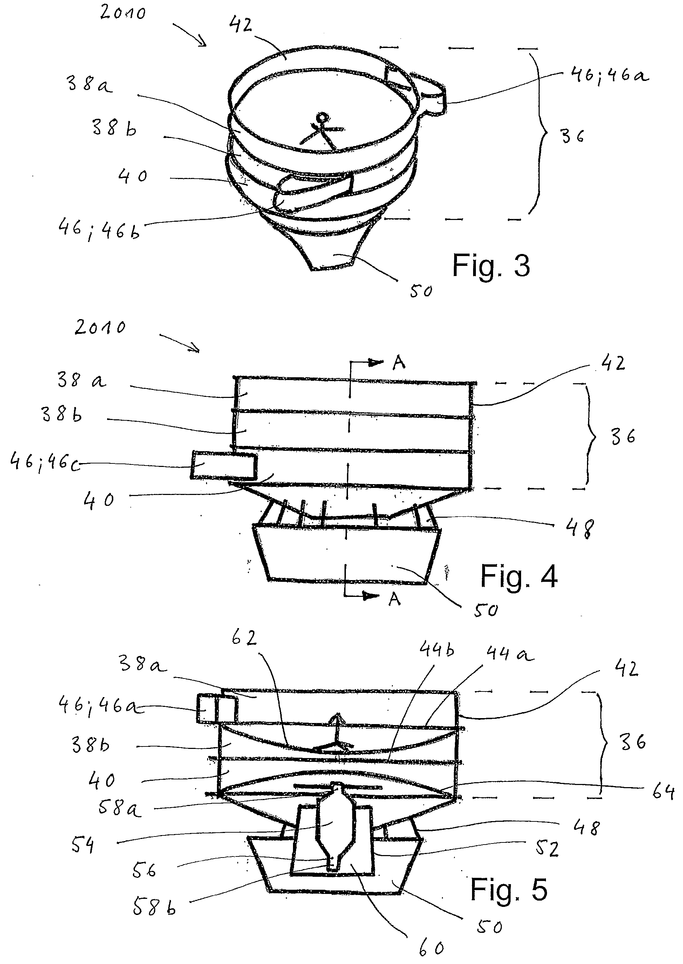

Ein für die Zwecke der vorliegenden Erfindung besonders gut geeigneter Feststoffabtrenner stellt das in den Fig. 3 bis 5 gezeigte Schwingsieb 201 dar.

Das Schwingsieb 2010 umfasst eine Siebeinheit 36 enthaltend zwei übereinander gestapelte Siebbehälter 38a, 38b, welche auf einem Siebdurchgangbehälter 40 angeordnet sind. Die Siebbehälter 38a, 38b und der Siebdurchgangbehälter 40 weisen einen im Wesentlichen kreisförmigen, identischen Querschnitt auf und sind nach aussen hin über eine in Umlaufrichtung verlaufende Mantelwand 42 begrenzt.

Wie insbesondere aus Fig. 5 hervorgeht, weist der von oben d.h. in Richtung des Siebens betrachtet erste Siebbehälter 38a einen ersten Siebbelag 44a auf, welcher eine grössere Maschenweite aufweist als der zweite Siebbelag 44b des darauffolgenden zweiten Siebbehälters 38b. Sowohl die Siebbehälter 38a, 38b als auch der Siebdurchgangbehälter 40 weist einen radial von der jeweiligen Mantelwand 42 abstehenden Auslass 46 auf.

Die Siebeinheit 36 ist über entsprechende Federelemente 48 auf einem Sockel 50 abgestützt. An der Siebeinheit 36 ist weiter ein Antriebsgehäuse 52 mit einem Antrieb 54 angeordnet, der eine Antriebswelle 56 aufweist, auf deren Endbereichen 58a, 58b jeweils eine exzentrische Schwungmasse 60 sitzt.

Während des Betriebs wird die Siebeinheit 36 somit in eine kreisförmige Schwingbewegung versetzt. Dadurch wird das in den ersten Siebbehälter 38a aufgegebene Presswasser ständig bewegt, wobei insbesondere auch die sich auf der Oberfläche des ersten Siebbelags 44a absetzenden Feststoffbestandteile des Presswassers ständig bewegt werden und ein Verstopfen des Siebbelags wirksam verhindert wird.

Der eine Grobfraktion an Feststoffbestandteilen enthaltende Siebüberstand wird sodann über den Grobfraktionsauslass 46a abgeführt, während der noch eine Feinfraktion an Feststoffbestandteilen enthaltende Siebdurchgang in einem unterhalb des ersten Siebbelags 44a angeordneten gewölbten Becken 62 aufgefangen und dem zweiten Siebbelag 44b zugeführt wird.

Aus dem Sieben im zweiten Siebbehälter 38b resultiert ein die Feinfraktion enthaltender Siebüberstand, welcher über den Feinfraktionsauslass 46b abgeführt wird, und eine Restflüssigkeit mit einem stark verminderten Trockensubstanzanteil von höchstens 15 Gew.-%. Diese Restflüssigkeit fliesst über einen nach oben gewölbten Boden des Siebdurchgangbehälters an dessen Rand, von wo sie letztendlich über den Restflüssigkeitsauslass 46c aus der Siebeinheit 36 abgeführt und in den Fermenter zurückgeführt wird.

### Referenzzeichenliste

- 1: Fermenter
- 2: Mischvorrichtung
- 3: Fermentereinlass
- 5: Digestatzuleitung
- 10: Fermentertank
- 11: Rührwerkantrieb
- 12: Speiseleitung
- 13: Entwässerungsvorrichtung
- 14: Fermenterauslass
- 15: Presswassertank
- 16;: Restflüssigkeitzuleitung;
- 160; 161: Filtratzuleitung; Siebdurchgangzuleitung
- 17: Dosierförderer
- 18: Austragseinheit
- 19: Förderband
- 20;: Feststoffabtrenner;
- 200;: Filtervorrichtung;
- 201; 2010: Sieb; Schwingsieb
- 21: erster Presswasserzuleitungsabschnitt
- 22: zweiter Presswasserzuleitungsabschnitt
- 20: Filtervorrichtung
- 24: Filtratzuleitung
- 30: Fermenterrückleitung
- 31: Schieber in Fermenterrückleitung
- 32: Schieber in Speiseleitung
- 33: Verbindungsleitung
- 34: Filtrattank
- 36: Siebeinheit
- 38a, 38b: Siebbehälter
- 40: Siebdurchgangbehälter
- 42: Mantelwind
- 44a, 44b: Siebbelag
- 46a, 46b, 46c: Auslass
- 48: Federelemente
- 50: Sockel
- 52: Antriebsgehäuse
- 54: Antrieb
- 56: Antriebswelle
- 58a, 58b: Endbereiche der Antriebswelle
- 60: exzentrische Schwungmasse
- 62: Becken
- 64: Boden des Siebdurchgangbehälters
- 101: einlassseitiges Ende des Fermentertanks
- 102: auslassseitiges Ende des Fermentertanks
- B: Fermentationsgut
- BS: Fermentationsgutsuspension
- D: Digestat
- PW: Presswasser
- PK: Presskuchen
- RF; F, SD: Restflüssigkeit; Filtrat, Siebdurchgang
- RS; R, SU: Restfeststoff; Retentat, Siebüberstand

## Patentansprüche

1. Verfahren zur anaeroben Fermentation von biogenen Abfallstoffen umfassend die Schritte, dass
a) ein die Abfallstoffe enthaltendes Fermentationsgut (B) mit Flüssigkeit gemischt wird und die derart erhaltene Fermentationsgutsuspension (BS) in einem Fermenter (1) anaerob fermentiert wird und
b) das bei der Fermentation der Fermentationsgutsuspension (BS) anfallende Digestat (D) mittels einer Entwässerungsvorrichtung (13) entwässert wird, wobei unter Abscheidung von Presswasser (PW) ein Presskuchen (PK) mit einem gegenüber dem Digestat (D) erhöhten Trockensubstanzanteil erhalten wird,
**dadurch gekennzeichnet, dass** das Verfahren die zusätzlichen Schritte umfasst, dass
c) das Presswasser (PW) einer Feststoffabtrennung unterzogen wird, wobei eine Restflüssigkeit (RF; F) mit einem gegenüber dem Presswasser (PW) reduzierten Trockensubstanzanteil von höchstens 15 Gew.-% erhalten wird und
d) die Restflüssigkeit (RF; F) mindestens teilweise für das Mischen gemäss a) verwendet wird.

2. Verfahren gemäss Anspruch 1, **dadurch gekennzeichnet, dass** es kontinuierlich oder quasi-kontinuierlich durchgeführt wird.

3. Verfahren gemäss Anspruch 1 oder 2, dass die Feststoffabtrennung gemäss Schritt c) mittels Filtration und/oder mittels Sieben, insbesondere unter Verwendung eines Schwingsiebs und/oder eines Rüttelsiebs, erfolgt und die Restflüssigkeit durch das Filtrat (F) der Filtration bzw. durch den Siebdurchgang des Siebens gebildet wird.

4. Verfahren gemäss einem der vorhergehenden Ansprüche, **dadurch gekennzeichnet, dass** das in Schritt b) erhaltene Presswasser (PW) und/oder die in Schritt c) erhaltene Restflüssigkeit (RF; F) vor dem jeweils nachfolgenden Schritt zwischengelagert wird.

5. Verfahren gemäss einem der vorhergehenden Ansprüche, **dadurch gekennzeichnet, dass** das Fermentationsgut (B) mit der Restflüssigkeit (RF; F) und optional weiterem Wasser (W) in einer Mischvorrichtung gemischt wird, bevor die derart erhaltene Fermentationsgutsuspension (BS) in den Fermenter (1) eingeführt wird.

6. Verfahren gemäss einem der vorhergehenden Ansprüche, **dadurch gekennzeichnet, dass** die Verdünnung des Fermentationsguts (B) gemäss Schritt a) derart durchgeführt wird, dass der Trockensubstanzanteil in der Fermentationsgutsuspension (BS) zwischen 25 und 35 Gew.-% beträgt.

7. Verfahren gemäss einem der vorhergehenden Ansprüche, wobei dem Fermentationsgut (B) oder der Fermentationsgutsuspension (BS) ein Teil des Digestats (D), des bei der Entwässerung gemäss Schritt b) erhaltenen Presskuchens (PK), des bei der Feststoffabtrennung in Schritt c) erhaltenen Restfeststoffs (RS; R) oder eine Mischung davon als Impfmaterial zugesetzt wird.

8. Verfahren gemäss einem der vorhergehenden Ansprüche, **dadurch gekennzeichnet, dass** einer oder mehrere Parameter aus der Gruppe bestehend aus:
- pH-Wert,
- Pufferkapazität,
- H₂S-Gehalt,
- Ammoniumionen- oder Ammoniak-Gehalt,
- Gehalt an Fettsäuren und
- Gehalt an kurzkettigen Carbonsäuren
der Restflüssigkeit (RF; F) bestimmt und auf einen gewünschten Wert eingestellt wird.

9. Verfahren gemäss einem der vorhergehenden Ansprüche, **dadurch gekennzeichnet, dass** der Restflüssigkeit (RF; F) folgende Hilfs- oder Zusatzstoffe allein oder im Gemisch beigegeben werden:
- Säure,
- Lauge,
- Puffer,
- Mikronährelemente oder Spurenelemente und
- Makronährelemente.

10. Verfahren gemäss einem der Ansprüche 3 bis 9, **dadurch gekennzeichnet, dass** das Sieben gemäss Schritt c) mittels eines Siebes, insbesondere eines Schwingsiebs und/oder eines Rüttelsiebs, mit zwei oder mehr Siebstufen erfolgt, wobei in einer ersten Siebstufe c1) eine Grobfraktion an Feststoffbestandteilen vom Presswasser abgetrennt wird, und von der aus der Stufe c1) als Siebdurchgang verbleibenden Suspension in einer nachfolgenden zweiten Siebstufe c2) eine Feinfraktion abgetrennt wird, um die Restflüssigkeit zu erhalten wird.

11. Anlage zur Durchführung des Verfahrens gemäss einem der vorhergehenden Ansprüche umfassend
A) einen Fermenter (1) zur Durchführung der anaeroben Fermentation und
B) eine mit dem Fermenter (1) über eine Digestatzuleitung (5) verbundene Entwässerungsvorrichtung (13) zur Entwässerung des Digestats (D),
**dadurch gekennzeichnet, dass** die Anlage zudem
C) einen mit der Entwässerungsvorrichtung (13) über eine Presswasserzuleitung (21, 22) verbundenen Feststoffabtrenner (20; 200, 201) zur Abtrennung von Restfeststoff aus dem in der Entwässerungsvorrichtung (13) erhaltenen Presswasser (PW), wobei der Feststoffabtrenner (20; 200, 201) derart ausgestaltet ist, dass die dabei erhaltene Restflüssigkeit einen Trockensubstanzanteil von höchstens 15 Gew.-% aufweist, und
D) eine Restflüssigkeitzuleitung (16; 160, 161) zur mindestens teilweisen Rückführung der Restflüssigkeit (RF; F, SD) in den Fermenter umfasst.

12. Anlage gemäss Anspruch 11, **dadurch gekennzeichnet, dass** der Feststoffabtrenner (20) eine Filtrationsvorrichtung (200) und/oder ein Sieb (201) ist, welche(s) derart ausgestaltet ist, dass das dabei erhaltene Filtrat (F) bzw. der dabei erhaltene Siebdurchgang (SD) einen Trockensubstanzanteil von höchstens 15 Gew.-% aufweist.

13. Anlage gemäss Anspruch 11 oder 12, **dadurch gekennzeichnet, dass** der Fermenter (1) wenigstens annähernd horizontal ausgerichtet ist.

14. Anlage gemäss einem der Ansprüche 11 bis 13, **dadurch gekennzeichnet, dass** die Restflüssigkeitszuleitung (16) in
E) eine Mischvorrichtung (2) zum Mischen des Fermentationsguts mit Flüssigkeit mündet, welche über eine Speiseleitung (12) mit dem Fermenter (1) verbunden ist.

15. Anlage gemäss einem der Ansprüche 12 bis 14, **dadurch gekennzeichnet, dass** das Sieb (201) ein Schwingsieb (2010) und/oder ein Rüttelsieb ist.
